Europäisches Patentamt

(19) European Patent Office

Office européen des brevets

(11) Publication number : **0 308 097 B1**

# (12) EUROPEAN PATENT SPECIFICATION

(45) Date of publication of patent specification :
**29.01.92 Bulletin 92/05**

(51) Int. Cl.$^5$ : **C07C 6/12**

(21) Application number : **88307983.2**

(22) Date of filing : **30.08.88**

(54) **Transalkylation of polyalkylaromatic hydrocarbons.**

(30) Priority : **02.09.87 US 92503**

(43) Date of publication of application :
**22.03.89 Bulletin 89/12**

(45) Publication of the grant of the patent :
**29.01.92 Bulletin 92/05**

(84) Designated Contracting States :
**BE DE FR GB IT NL**

(56) References cited :
**EP-A- 0 141 514**

(73) Proprietor : **MOBIL OIL CORPORATION**
**150 East 42nd Street**
**New York New York 10017 (US)**

(72) Inventor : **Chu, Yung-Feng**
**4 Parker Road**
**Plainsboro New Jersey 08536 (US)**
Inventor : **Marler, David Owen**
**801 Cooper Street No. 272B**
**Deptford New Jersey 08096 (US)**
Inventor : **McWilliams, John Paul**
**117 South American Street**
**Woodbury New Jersey 08096 (US)**

(74) Representative : **Colmer, Stephen Gary**
**Patent Department c/o Mobil Services**
**Company Limited Mobil Court 3 Clements Inn**
**London WC2A 2EB (GB)**

## Description

This invention is directed to a process for transalkylating a polyalkylaromatic hydrocarbon charge to prepare monoalkylaromatics in the presence of a catalyst.

Alkylation of aromatic hydrocarbons utilizing porous crystalline silicate catalysts has heretofore been described, U.S. Pat. No. 2,904,607 to Mattox refers to alkylation of aromatic hydrocarbons with an olefin in the presence of a crystalline metallic aluminosilicate having uniform pore openings of about 6 to 15 Angstrom units. U.S. Pat. No. 3,251,897 to Wise describes liquid phase alkylation in the presence of X- or Y-type crystalline aluminosilicate zeolite, specifically such type zeolites wherein the cation is rare earth and/or hydrogen. U.S. Pat. No. 3,751,504 to Keown et al., and U.S. Pat. No. 3,751,506 to Burress describe vapor phase alkylation of aromatic hydrocarbons with olefins e.g., benzene with ethylene, in the presence of ZSM-5 zeolite catalyst. U.S. Pat. No. 4,016,218 to Wise describes vapor phase alkylation with a shape-selective zeolite such as ZSM-5 which has been modified by steaming to an alpha value less than 250. U.S. Patent No. 4,169,111 to Wight describes alkylation of benzene with ethylene wherein polyethylbenzene by-products can be subjected to transalkylation with benzene in a separate transalkylation zone to produce ethylbenzene using a zeolite catalyst having a $SiO_2/Al_2O_3$ ratio of between 2 and 80 ; e.g. zeolite Y, mordenite or ZSM-5.

EP-A-0141514 discloses a process for converting alkylbenzene compounds to a mixture of dialkylbenzenes in the presence of a catalyst composite comprising a crystalline zeolite material and a matrix material.

During the alkylation of alkylatable aromatic compounds, undesirable polyalkylaromatics can be formed which can be recycled to the alkylation reactor where further conversion to monoalkylaromatics can occur. However, polyalkylaromatics can cause the formation of heavy aromatic residues and enhance catalyst aging in the alkylation reactor. Accordingly, polyalkylaromatics can be removed to a separate transalkylation reactor having the same reactor conditions and catalyst as the alkylation reactor, as is taught by U.S. Patent No. 3,751,504 to Keown et al. This reference teaches the use of ZSM-5 having a $SiO_2/Al_2O_3$ molar ratio of 5 to 300, preferably 5 to 100.

It has now been found that transalkylation is preferably carried out under conditions dissimilar to those employed during alkylation. In particular, it has been found that transalkylation is thermodynamically favored at lower temperatures while using catalysts of higher acid activity than those employed in alkylation. In particular, it has been found that higher acidity zeolites evidence increased selectivity toward monoalkylaromatic product. Moreover, certain of these catalysts, e.g. those having a constraint index of 5 to 9, e.g. ZSM-5 and ZSM-11 are capable of greater benzene retention. Greater benzene retention reduces the amount of benzene required for transalkylation, permitting more economical operation.

This invention contemplates a process for effecting transalkylation of polyalkylaromatics which comprises contacting a polyalkylaromatic hydrocarbon charge with benzene under conditions effective for accomplishing said transalkylation, including a reactor temperature between 204 and 538°C (400°F and 1,000°F), preferably less than 343°C (650°F) say, between 260 and 454°C (500 and 850°F), a pressure between 101.3 to 20,790 kPa (0 and 3000 psig), preferably between 276 and 5275 kPa (25 and 750 psig), a total feed weight hourly space velocity (WHSV) between 0.1 hr$^{-1}$ and 100 hr$^{-1}$, preferably between 2 and 50 hr$^{-1}$, and a hydrogen to hydrocarbon mole ratio of from 0 to 4, preferably from 0 to 2, with a catalyst composition comprising a crystalline molecular sieve having a high lattice aluminum content characterized by a silica/alumina mole ratio of less than 40, preferably from 20 to 30, a Constraint Index of from 5 to 9. The catalyst composition has an alpha value of at least 140, preferably at least 200. The above WHSV is based upon the weight of catalyst molecular sieve, i.e. total weight of active catalyst component.

In one aspect, the present invention relates to a process for reducing the polyalkylbenzene content, of e.g. polyethylbenzene, to a polyalkylaromatic effluent by cofeeding the effluent with benzene to a catalytic transalkylation zone and recovering monoalkylbenzene, e.g., ethylbezene.

The present invention relates to a transalkylation process for polyalkylaromatics. U.S. Patent 4,169,111 is illustrative of a prior art process for transalkylation of polyalkylaromatics over a wide range of conditions and with a catalyst composition comprising a crystalline molecular sieve characterized by a silica/alumina mole ratio of between 2 and 80, e.g., X, Y, L, B, ZSM-5 and Omega Crystal types.

For the present improved process, the catalyst will comprise a crystalline molecular sieve material having a structure which will permit a Constraint Index of from 5 to 9. The silica/alumina mole ratio of the molecular sieve for use herein, however, should be less than 40, preferably from 20 to less than 30, e.g. from 20 to 28. The alpha value of the catalyst employed in the presently claimed process should be at least 140, preferably 200 to 3500 or even more preferably greater than 500, say from 500 to 3000. A definition of alpha value and technique employed in its determination are given below.

The crystalline molecular sieves which can be made to exhibit the above required properties include those having the structure of ZSM-5, ZSM-11, ZSM-5/ZSM-11 intermediate, ZSM-12, ZSM-22, ZSM-23, ZSM-35, ZSM-48, ZSM-50 and Beta, with zeolites having a Constraint Index between 5 and 9 such as ZSM-5 and ZSM-11, being particularly preferred owing to their enhanced benzene retention properties. ZSM-5 is described in U.S. Patent 3,702,886 ; ZSM-11 is described in U.S. Patent 3,709,979 ; ZSM-5/ZSM-11 intermediate is described in U.S. Patent 4,229,424 ; ZSM-12 is described in U.S. Patent 3,832,449 ; ZSM-22 is described in U.S. Patent 4,556,477 ; ZSM-23 is described in U.S. Patent 4,076,842 ; ZSM-35 is described in U.S. Patent 4,106,245 ; ZSM-48 is described in U.S. Patent 4,397,827 ; ZSM-50 is described in U.S. Patent 4,640,849 ; Beta is described in U.S. Patent 3,308,069.

The original cations of the above molecular sieves are preferably replaced in accordance with techniques well known in the art, at least in part, with hydrogen or hydrogen precursor cations and/or non-noble metal ions of Group VIII of the Periodic Table, e.g. nickel, iron and/or cobalt.

A convenient measure of the extent to which a crystal provides access to molecules of varying sizes to it internal structure is the Constraint Index of the crystal. Crystalline materials which provide a highly restricted access to and egress from its internal structure have a high value for the Constraint Index, and materials of this kind usually have pores of small size, e.g. less than 5 Angstroms. On the other hand, crystalline materials which provide relatively free access to the internal crystal structure have a low value for the Constraint Index, and usually pores of large size, e.g. greater than 8 Angstroms. The method by which Constraint Index is determined is described fully in U.S. Patent 4,106,218.

Constraint Index (CI) valves for some typical materials are :

| | CI | (at test temperature) |
|---|---|---|
| ZSM-4 | 0.5 | (316°C) |
| ZSM-5 | 6-8.3 | (371°C - 316°C) |
| ZSM-11 | 5-8.7 | (371°C - 316°C) |
| ZSM-12 | 2.3 | (316°C) |
| ZSM-20 | 0.5 | (371°C) |
| ZSM-22 | 7.3 | (427°C) |
| ZSM-23 | 9.1 | (427°C) |
| ZSM-34 | 50 | (371°C) |
| ZSM-35 | 4.5 | (454°C) |
| ZSM-48 | 3.5 | (538°C) |
| ZSM-50 | 2.1 | (427°C) |
| TMA Offretite | 3.7 | (316°C) |
| TEA Mordenite | 0.4 | (316°C) |
| Clinoptilolite | 3.4 | (510°C) |
| Mordenite | 0.5 | (316°C) |
| REY | 0.4 | (316°C) |
| Amorphous Silica-alumina | 0.6 | (538°C) |
| Dealuminized Y | 0.5 | (510°C) |
| Erionite | 38 | (316°C) |
| Zeolite Beta | 0.6-2.0 | (316°C-399°C) |

The molecular sieve for use herein or the catalyst comprising same can be thermally treated at high temperatures. This thermal treatment is generally performed by heating at a temperature of at least 370°C for a least 1 minute and generally not longer than 20 hours. While subatmospheric pressure can be employed for the thermal treatment, atmospheric pressure is desired for reasons of convenience. The thermal treatment can be performed at a temperature up to 925°C. The thermally treated product is particularly useful in the present process.

For the transalkylation process of this invention the suitable molecular sieve may be employed in combination with a support or binder material such as, for example, a porous inorganic oxide support or a clay binder. Non-limiting examples of such binder materials include alumina, zirconia, silica, magnesia, thoria, titania, boria and combinations thereof, generally in the form of dried inorganic oxide gels and gelatinous precipitates. Suitable clay materials include, by way of example, bentonite and kieselguhr. The relative proportion of suitable crystalline molecular sieve of the total composition of catalyst and binder or support may vary widely with the zeolite content ranging from between 30 to 90 percent by weight and more usually in the range of 50 to 80 percent by weight of the composition. The composition may be in the form of an extrudate, beads or fluidizable microspheres.

Operating conditions employed in the process of the present invention are critical. Such conditions as temperature, pressure, space velocity, molar ratio of the reactants and hydrogen to hydrocarbon mole ratio will have important effects on the process.

The process of this invention is conducted such that alkylation of polyalkylaromatics is carried out by contact in a reaction zone, such as, for example, a fixed bed of catalyst composition, under transalkylation effective conditions, said catalyst composition being characterized as comprising the above-defined molecular sieve, preferably which has been hydrogen, or hydrogen precursor exchanged and/or thermally treated. The effluent is separated and distilled to remove desired product, such as monoalkylaromatics, and unreacted reactants, benzene and polyalkylaromatics are recycled for further reaction.

By the present improved process polyalkylaromatics such as triethylbenzene and diethylbenzene can be converted to aromatic concentrates of high value, e.g. ethylbenzene. This process may be conducted in either batch or fluid bed operation with attendant benefits of either operation readily obtainable.

Typical feedstocks are those containing 40 to 80 wt.% benzene, 15 to 50 wt.% dialkylbenzene, and 2 to 20 wt.% $C_9$ monoalkylaromatics and 0 to 5 wt.% $C_{9+}$ aromatics.

The following specific examples will serve to illustrate the process of the present invention, without unduly limiting same. In the examples, when Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of the highly active silica-alumina cracking catalyst taken as an Alpha of 1 (Rate Constant = 0.016 sec$^{-1}$). The Alpha Test is described in U.S. Patent 3,354,078 and in The Journal of Catalysis, Vol. IV, pp. 522-529 (August 1965). It is noted that intrinsic rate constants for many acid-catalyzed reactions are proportional to the Alpha Value for a particular crystalline silicate catalyst (see "The Active Site of Acidic Aluminosilicate Catalysts," Nature, Vol. 309, No. 5959, pp. 589-591, 14 June 1984).

Example 1

Three separate molecular sieves were prepared for testing of the present concept and comparisons to determine important process/catalyst limitations.

MOLECULAR SIEVE A

For the synthesis of the crystals a 7.3 parts quantity by weight of water was mixed with 12.8 parts, 50% NaOH, 10.1 parts $Al_2(SO_4)_3$ 14$H_2O$, 1.6 parts ZSM-5 seeds and 68.2 parts amorphous silica (047.6% solids) prepared by the neutralization of sodium silicate with sulfuric acid. The reaction mixture had a composition in mole ratios of :

$SiO_2/Al_2O_3$     32
$H_2O/SiO_2$     5.45
$OH^-/SiO_2$     0.105
$OH^-/H_2O$     0.0192

The reaction mixture was heated directly to 104°C (220°F) and stirred in an autoclave at that temperature for crystallization. After full crystallinity was achieved, the resulting crystals were separated from remaining liquid by filtration, washed with water and dried.

MOLECULAR SIEVE B

A 15.0 parts quantity by weight of water was mixed with 11.0 parts 50% NaOH, 9.0 parts $Al_2(SO_4)_3 \cdot 14$ $H_2O$ and 65.0 parts amorphous silica (42.7% solids) prepared by the neutralization of sodium silicate with sulfuric acid. The reaction mixture had a composition in mole ratios of :

| | |
|---|---|
| $SiO_2/Al_2O_3$ | 32 |
| $H_2O/SiO_2$ | 7.04 |
| $OH^-/SiO_2$ | 0.104 |
| $OH^-/H_2O$ | 0.015 |

The reaction mixture was then heated to 177°C (350°F) and stirred in an autoclave at that temperature for crystallization. After full crystallinity was achieved, the resulting crystals were separated from remaining liquid by filtration, washed with water and dried.

The crystals were then formed into an extrudate containing 35 weight percent alumina. The extrudate was exchanged with NaCl, steamed at 101 : 3 kPa (0 psig) and 100% steam at 371°C (700°F) for 6 h, and then calcined at 538°C (1000F) for 3 h in air.

MOLECULAR SIEVE C

A 2.4 parts quantity by weight of tripropylamine was added to a mixture containing 2.1 parts n-propylbromide, 4.0 parts methylethylketone, 6.9 parts sodium chloride, 0.1 part dispersant (mixture of polymerized aryl and substituted benzoid alkyl sulfonic acids), 3.2 parts 93% $H_2SO_4$, 1.3 parts $Al_2(SO_4)_3 \cdot 14\,H_2O$, 37 percent sodium silicate (28.5% $SiO_2$, 8.8% $Na_2O$) and 43 parts water. The reaction mixture has a composition of the following molar ratios :

| | |
|---|---|
| $SiO_3/Al_2O_3$ | 78 |
| $H_2O/SiO_2$ | 21.09 |
| $OH^-/SiO_2$ | 0.087 |
| $N/Al_2O_3$ | 7.57 |

where N is tripropylamine and the hydroxide concentration is based only on inorganic sources.

The reaction mixture was then heated directly to 104°C (220°F) and stirred in an autoclave at that temperature for crystallization. After full crystallinity was achieved, the resulting crystals were separated from the remaining liquid by filtration, washed with water and dried.

The above molecular sieve materials were evaluated for composition, e.g. alumina, silica and sodium contents, surface area, particle density, pore volume and Alpha Value. All had sodium contents of less than 500 ppm. Other results of these evaluations are listed in Table I below.

## TABLE I

| Molecular Sieve | A | B | C |
|---|---|---|---|
| $SiO_2/Al_2O_3$, mole ratio | 25 | 25* | 70 |
| Surface area, $m^2/g$ | 333 | 325 | -- |
| Particle density, g/cc | 1.01 | 0.95 | -- |
| Pore volume, cc/g | 0.60 | 0.67 | -- |
| Alpha Value | 600 | 3000 | 800 |

*before steaming

### Example 2

The molecular sieves of Example 1 were each composited with binder alumina and made into extrudates such that each catalyst comprised 65 wt.% of its Molecular Sieve component and 35 wt.% alumina. Extrudate containing Molecular Sieve B was steamed as set out in Example 1.

Each catalyst was then evaluated for transalkylation in identical reactors and at identical reaction conditions. The reactors were 0.95 cm (3/8-inch) o.d. stainless steel and the reaction conditions were 101.3 kPa (0 psig), 5 hr$^{-1}$ weight hourly space velocity (based on molecular sieve) and a hydrogen/hydrocarbon mole ratio of 0. Feedstock was 29.6% diethylbenzene, 50.7% benzene 9.1% n-propylbenzene.

Liquid and gas products from the reactions were analyzed by conventional chromatography. Selectivity is defined as weight percent gain of ethylbenzene divided by weight percent losses of diethylbenzene and benzene. Run data are presented in Table II below.

It will be appreciated that the operating conditions for the reaction in accordance with the process of this invention, as exemplified in the foregoing examples, may be varied within the limits specified so that the process may be conducted in vapor-phase.

### TABLE II

| Molecular Siev | Diethylbenzene Wt% Loss | Benzene Wt% Loss | Ethylbenzene Wt% Gain | Selectivity | Alpha Value (Catalyst) |
|---|---|---|---|---|---|
| A | 13.1 | 5.7 | 25.1 | 134 | 600 |
| B | 12.9 | 9.4 | 30.7 | 138 | 3000 |
| C | 5.9 | 12.2 | 22.2 | 123 | 200 |

Transalkylation 316°C (600°F), WHSV 5, Selectivity $= \dfrac{\text{Ethylbenzene wt\% gain}}{\text{Diethylbenzene wt\% loss + Benzene wt\% loss}} \times 100$

EP 0 308 097 B1

## Claims

1. A process for effecting transalkylation of a polyalkylaromatic hydrocarbon charge to prepare a monoalkylaromatic which comprises contacting said charge with benzene under conditions effective for accomplishing said transalkylation in the presence of a catalyst composition comprising a zeolite having a constraint index from 5 to 9, characterized by a silica/alumina mole ratio of less than 40 and an alpha value of at least 140.

2. The process of claim 1 wherein said zeolite has a silica/alumina mole ratio of from 20 to 30.

3. The process of claim 1 wherein said zeolite has an alpha value of 500 to 3000.

4. The process of claim 1 wherein said zeolite has the structure of ZSM-5.

5. The process of claim 1 wherein said zeolite has the structure of ZSM-11.

6. The process of claim 1 wherein said zeolite contains cations selected from the group consisting of hydrogen and hydrogen precursor.

7. The process of claim 1 wherein said conditions effective for accomplishing said transalkylation include a temperature of less than 343°C (650°F), a pressure of from 101.3 to 20,790 kPa (0 to 3000 psig), a hydrogen/hydrocarbon mole ratio of 0 to 4 and a weight hourly space velocity, based upon weight of active catalyst component, of from 0.1 hr$^{-1}$ to 100 hr$^{-1}$.

8. The process of claim 1 wherein said catalyst composition comprises said zeolite and a binder.

9. The process of claim 6 wherein said polyalkylaromatic hydrocarbon charge comprises dialkylbenzene.

10. The process of claim 15 wherein said polyalkylaromatic hydrocarbon charge comprises diethylbenzene and said monoalkylaromatic is ethylbenzene.

## Patentansprüche

1. Verfahren zur Durchführung der Transalkylierung einer polyalkylaromatischen Kohlenwasserstoffbeschickung, um Monoalkylaromaten herzustellen, das den Kontakt der Beschickung mit Benzol bei Bedingungen, die für die Durchführung dieser Transalkylierung effektiv sind, in Gegenwart einer Katalysatorzusammensetzung umfaßt, die einen Zeolith mit einem Zwangsindex von 5 bis 9 umfaßt, gekennzeichnet durch ein Siliciumdioxid/Aluminiumoxid-Molverhältnis von weniger als 40 und einen α-Wert von mindestens 140.

2. Verfahren nach Anspruch 1, worin der Zeolith ein Siliciumdioxid/Aluminiumoxid-Molverhältnis von 20 bis 30 aufweist.

3. Verfahren nach Anspruch 1, worin der Zeolith einen α-Wert von 500 bis 3000 aufweist.

4. Verfahren nach Anspruch 1, worin der Zeolith die Struktur von ZSM-5 aufweist.

5. Verfahren nach Anspruch 1, worin der Zeolith die Struktur von ZSM-11 aufweist.

6. Verfahren nach Anspruch 1, worin der Zeolith Kationen enthält, die aus der Gruppe ausgewählt sind, die aus Wasserstoff und einer Wasserstoffvorstufe besteht.

7. Verfahren nach Anspruch 1, worin die Bedingungen, die zur Durchführung der Transalkylierung effektiv sind, eine Temperatur von weniger als 343°C (650°F), einen Druck von 101,3 bis 20.790 kPa (0 bis 3000 psig), ein Wasserstoff/Kohlenwasserstoff-Molverhältnis von 0 bis 4 und eine stündliche Gewichts-Raum-Geschwindigkeit, bezogen auf das Gewicht der aktiven Katalysatorkomponente, von 0,1 h$^{-1}$ bis 100 h$^{-1}$ umfassen.

8. Verfahren nach Anspruch 1, worin die Katalysatorzusammensetzung den Zeolith und ein Bindemittel umfaßt.

9. Verfahren nach Anspruch 6, worin die polyalkylaromatische Kohlenwasserstoffbeschickung Dialkylbenzol umfaßt.

10. Verfahren nach Anspruch 15, worin die polyalkylaromatische Kohlenwasserstoffbeschickung Diethylbenzol umfaßt und die monoalkylaromatische Verbindung Ethylbenzol ist.

## Revendications

1. Un procédé de transalkylation d'une charge d'hydrocarbures polyalkylaromatiques afin de préparer un dérivé monoalkylaromatique consistant à mettre en contact ladite charge avec du benzène dans des conditions efficaces pour accomplir ladite transalkylation en présence d'une composition catalytique comprenant une zéolite ayant un indice de contrainte de l'ordre de 5 à 9, caractérisé par un rapport molaire silice/alumine inférieur à 40 et une valeur alpha d'au moins 140.

2. Le procédé selon la revendication 1, dans lequel ladite zéolite a un rapport molaire silice/alumine de l'ordre de 20 à 30.

7

3. Le procédé selon la revendication 1, dans lequel ladite zéolite a une valeur alpha de l'ordre de 500 à 3000.

4. Le procédé selon la revendication 1, dans lequel ladite zéolite a la structure d'une ZSM-5.

5. Le procédé selon la revendication 1, dans lequel ladite zéolite a la structure d'une ZSM-11.

6. Le procédé selon la revendication 1, dans lequel ladite zéolite contient des cations choisis parmi le groupe comprenant l'hydrogène et les précurseurs d'hydrogène.

7. Le procédé selon la revendication 1, dans lequel lesdites conditions efficaces pour mettre en oeuvre ladite transalkylation comprennent une température inférieure à 343°C (650°F), une pression de l'ordre de 101,3 à 20 790 kPa (0 à 3000 psig), un rapport molaire hydrogène/hydrocarbure de 0 à 4 et une vitesse horaire spatiale pondérale basée sur le poids du composant catalytique actif de 0,1 h$^{-1}$ à 100 h$^{-1}$.

8. Le procédé selon la revendication 1, dans lequel ladite composition catalytique comprend ladite zéolite et un liant.

9. Le procédé selon la revendication 6, dans lequel ladite charge d'hydrocarbures polyalkylaromatiques comprend du dialkylbenzène.

10. Le procédé selon la revendication 15, dans lequel ladite charge d'hydrocarbures polyalkylaromatiques consiste en diéthylbenzène et ledit dérivé monoalkylaromatique est de l'éthylbenzène.